# FASCICULE DE BREVET EUROPEEN

(11) **EP 0 770 070 B1**
(45) Date de publication et mention de la délivrance du brevet: **30.09.1998**
(21) Numéro de dépôt: 95925022.6
(22) Date de dépôt: 07.07.1995
(51) Int. Cl.: C07D 305/14, A61K 31/335

(54) **PROCEDE DE PREPARATION DU TRIHYDRATE DU (2R,3S)-3-TERT-BUTOXYCARBONYLAMINO-2-HYDROXY-3-PHENYLPROPIONATE DE 4-ACETOXY-2ALPHA-BENZOYLOXY-5BETA,20-EPOXY-1,7BETA,10BETA-TRIHYDROXY-9-OXO-TAX-11-EN-13ALPHA-YLE**
VERFAHREN ZUR HERSTELLUNG VON TRIHYDRAT VON (2R,3S)-3-TERT-BUTOXYCARBONYL-AMINO-2-HYDROXY-3-PHENYLPROPIONYLSÄURE ESTER VON 4-ACETOXY-2ALFA-BENZOYLOXY-5BETA, 20-EPOXY-1,7BETA, 10BETA-TRIHYDROXY-9-OXO-TAX-11-EN-13ALFA-YL
METHOD FOR PREPARING 4-ACETOXY-2ALPHA-BENZOYLOXY-5ALPHA,20-EPOXY-1,7BETA,10BETA-TRIHYDROXY-9-OXO-TAX-11-EN-13ALPHA-YL(2R,3S)-3-TERT-BUTOXYCARBONYLAMINO-2-HYDROXY-3-PHENYLPROPIONATE TRIHYDRATE

(30) Priorité: 08.07.1994 FR 9408479
(43) Date de publication de la demande: 02.05.1997
(73) Titulaire: RHONE-POULENC RORER S.A., 92160 Antony (FR)
(72) Inventeur: AUTHELIN, Jean-René, F-91180 Saint-Germain-les-Arpajon (FR); DOVEZE, Jacques, F-94130 Vauhallan (FR); FOUQUE, Elie, F-94100 Saint Maur des Fosses (FR); MANDARD, Bernadette, F-41110 Mareuil-sur-Cher (FR); TAILLEPIED, Isabelle, F-94700 Maisons-Alfort (FR)
(74) Mandataire: Le Pennec, Magali
(86) Numéro de dépôt international: FR9500910
(87) Numéro de publication internationale: WO9601815

(56) Documents cités:
- EP-A- 0 253 738
- CHEMICAL ABSTRACTS, vol. 113, no. 75, 1990, Columbus, Ohio, US; abstract no. 142641k, GUERITTE-VOGELEIN 'STRUCTURE OF A SYNTHETIC TAXOL PRECURSOR.' page 723 ; & ACTA CRYSTALLOGR. SECT. C:CRYST. STRUCT. COMMUN., vol.C46, no.5, 1990, ENGL. pages 781 - 784

## Description

La présente invention concerne un procédé de préparation du trihydrate de (2R,3S)-3-tert-butoxycarbonylamino-2-hydroxy-3-phénylpropionate de 4-acétoxy-2a-benzoyloxy-5β,20-époxy-1,7β,10β-trihydroxy-9-oxo-tax-11-èn-13α-yle.

Dans les brevets européens EP-0 253 738 et EP-0 336 841 sont décrits le (2R,3S)-3-tert-butoxycarbonylamino-2-hydroxy-3-phénylpropionate de 4-acétoxy-2a-benzoyloxy-5β,20-époxy-1,7β,10β-trihydroxy-9-oxo-tax-11-èn-13α-yle qui présente des propriétés anticancéreuses et antileucémiques remarquables, et sa préparation.

Il a été trouvé que le trihydrate du (2R,3S)-3-tert-butoxycarbonylamino-2-hydroxy-3-phénylpropionate de 4-acétoxy-2α-benzoyloxy-5β,20-époxy-1,7β,10β-trihydroxy-9-oxo-tax-11-èn-13α-yle présente une stabilité très nettement supérieure à celle du produit anhydre.

Selon l'invention, le trihydrate du (2R,3S)-3-tert-butoxycarbonylamino-2-hydroxy-3-phénylpropionate de 4-acétoxy-2α-benzoyloxy-5β,20-époxy-1,7β,10β-trihydroxy-9-oxo-tax-11-èn-13α-yle peut être obtenu après cristallisation du (2R,3S)-3-tert-butoxycarbonylamino-2-hydroxy-3-phénylpropionate de 4-acétoxy-2α-benzoyloxy-5β,20-époxy-1,7β,10β-trihydroxy-9-oxo-tax-11-èn-13α-yledans un mélange d'eau et d'un alcool aliphatique contenant 1 à 3 atomes de carbone suivi du séchage du produit obtenu dans des conditions déterminées de température, de pression et d'humidité.

Pour la mise en oeuvre du procédé selon l'invention, il peut être particulièrement avantageux
- de dissoudre le (2R,3S)-3-tert-butoxycarbonylamino-2-hydroxy-3-phénylpropionate de 4-acétoxy-2α-benzoyloxy-5β,20-époxy-1,7β,10β-trihydroxy-9-oxo-tax-11-èn-13α-yle, préalablement purifié par chromatographie, dans un alcool aliphatique contenant 1 à 3 atomes de carbone à une température comprise de préférence entre 40 et 60°C,
- d'éliminer éventuellement les solvants résiduels de chromatographie par co-distillation sous pression réduite, en remplaçant le volume de solvant distillé par de l'alcool pur,
- d'ajouter de l'eau éventuellement purifiée à la même température,
- puis après amorçage éventuel de la cristallisation et refroidissement à une température voisine de 0°C, de séparer les cristaux obtenus de trihydrate de (2R,3S)-3-tert-butoxycarbonylamino-2-hydroxy-3-phénylpropionate de 4-acétoxy-2α-benzoyloxy-5β,20-époxy-1,7β,10β-trihydroxy-9-oxo-tax-11-èn-yle-13α ainsi obtenus, puis de les sécher sous pression réduite en atmosphère à humidité contrôlée.

Généralement, le (2R,3S)-3-tert--butoxycarbonylamino-2-hydroxy-3-phénylpropionate de 4-acétoxy-2α-benzoyloxy-5β,20-époxy-1,7β,10β-trihydroxy-9-oxo-tax-11-èn-yle-13α purifié est dissous dans un excès de l'alcool aliphatique. De préférence, la quantité d'alcool est comprise entre 8 et 12 parties en poids par rapport au (2R,3S)-3-tert-butoxycarbonylamino-2-hydroxy-3-phénylpropionate de 4-acétoxy-2α-benzoyloxy-5β,20-époxy-1,7β,10β-trihydroxy-9-oxo-tax-11-èn-yle-13α mis en oeuvre.

Généralement, la distillation de l'alcool est effectuée sous pression réduite à une température voisine de 40°C jusqu'à l'obtention d'un sirop épais dont l'agitation est difficile. Il peut être avantageux de répéter plusieurs fois cette opération qui conduit à l'élimination des solvants résiduels contenus dans le produit purifié mis en oeuvre.

L'élimination des solvants résiduels étant terminée, le sirop obtenu est repris par une quantité d'alcool comprise entre 3,5 et 6 parties en poids par rapport au (2R,3S)-3-tert-butoxycarbonylamino-2-hydroxy-3-phénylpropionate de 4-acétoxy-2α-benzoyloxy-5 β,20-époxy-1,7β,10β-trihydroxy-9-oxo-tax-11-èn-13α-yle.

Après séparation éventuelle d'impuretés insolubles par filtration, de l'eau, de préférence purifiée, est ajoutée de façon telle que le rapport pondéral eau/alcool soit voisin de 2/1.

La cristallisation est amorcée puis le mélange est refroidi lentement jusqu'à une températurs voisine de 0°C.

Le trihydrate de (2R,3S)-3-tert-butoxycarbonylamino-2-hydroxy-3-phénylpropionate de 4-acétoxy-2α-benzoyloxy-5β,20-époxy-1,7β,10β-trihydroxy-9-oxo-tax-11-èn-13α-yle qui cristallise est séparé, de préférence par filtration ou centrifugation, puis séché. Le séchage est effectué sous pression réduite, comprise ente 4 et 7 kPa, à une température voisine de 40°C dans une atmosphère à humidité contrôlée, l'humidité relative étant voisine de 80 %.

Pour la mise en oeuvre du procédé, il peut être avantageux d'effectuer la cristallisation en présence d'acide ascorbique qui est ajouté lors de la dissolution du (2R,3S)-3-tert-butoxycarbonylamino-2-hydroxy-3-phénylpropionate de 4-acétoxy-2α-benzoyloxy-5β,20-époxy-1,7β,1 0β-trihydroxy-9-oxo-tax-11-èn-13α-yle purifié dans l'alcool. Il est possible d'utiliser jusqu'à 1 % en poids d'acide ascorbique.

Pour la mise en oeuvre du procédé, il est particulièrement avantageux d'utiliser l'éthanol comme alcool.

La structure trihydrate du (2R,3S)-3-tert-butoxycarbonylamino-2-hydroxy-3-phénylpropionate de 4-acétoxy-2α-benzoyloxy-5β,20-époxy-1,7β,10β-trihydroxy-9-oxo-tax-11-èn-13α-yle est confirmée par diffraction X, par analyse thermogravimétrique et par analyse calorimétrique différentielle.

Plus particulièrement, l'analyse thermogravimétrique montre une perte de masse entre 40 et 140°C de 6,1 %, ce qui correspond à trois molécules d'eau pour une molécule de (2R,3S)-3-tert-butoxycarbonylamino-2-hydroxy-3-phénylpropionate de 4-acétoxy-2α-benzoyloxy-5β,20-époxy-1,7β,10β-trihydroxy-9-oxo-tax-11-èn-13α-yle.

La méthode de dosage de l'eau massique et de l'eau d'hydratation par analyse calorimétrique différentielle montre l'absence d'eau massique et un signal endothermique à 132,6°C correspond à la dissociation d'un hydrate.

Le trihydrate de (2R,3S)-3-tert-butoxycarbonylamino-2-hydroxy-3-phénylpropionate de 4-acétoxy-2a-benzoyloxy-5β,20-époxy-1,7β,10β-trihydroxy-9-oxo-tax-11-èn-13α-yle présente la propriété de ne plus posséder de caractère hygroscopique.

Les études de stabilité montrent que le trihydrate de (2R,3S)-3-tert-butoxycarbonylamino-2-hydroxy-3-phénylpropionate de 4-acétoxy-2α-benzoyloxy-5β,20-époxy-1,7β,10β-trihydroxy-9-oxo-tax-11-èn-13α-yle est stable à 4°C, 25°C et 35°C dans une atmosphère à 90 % d'humidité relative jusqu'à 18 mois sans modification de sa forme cristalline.

Dans les mêmes conditions, le (2R,3S)-3-tert-butoxycarbonylamino-2-hydroxy-3-phénylpropionate de 4-acétoxy-2α-benzoyloxy-5β,20-époxy-1,7β,10β-trihydroxy-9-oxo-tax-11-èn-13α-yle anhydre, dont la forme cristalline est différente, évolue lentement vers la forme trihydratée.

Les exemples suivants illustrent la présente invention.

### EXEMPLE 1

Dans un réacteur à l'abri de la lumière, on introduit 303 g de (2R,3S)-3-tert-butoxycarbonylamino-2-hydroxy-3-phénylpropionate de 4-acétoxy-2a-benzoyloxy-5β,20-époxy-1,7β,10β-trihydroxy-9-oxo-tax-11-èn-13α-yle dont le titre est de 92,4 % (0,314 mole) et 2,875 kg d'éthanol absolu (d = 0,79). Le mélange est chauffé à 40°C jusqu'à dissolution complète du (2R,3S)-3-tert-butoxycarbonylamino-2-hydroxy-3-phénylpropionate de 4-acétoxy-2α-benzoyloxy-5β,20-époxy 1,7β,10β-trihydroxy-9-oxo-tax-11-èn-13α-yle. On distille alors l'éthanol sous une pression voisine de 12 kPa jusqu'à l'obtention d'un sirop à la limite d'agitation. On ajoute au sirop 0,983 kg d'éthanol et on distille à nouveau dans les mêmes conditions. Au sirop obtenu, on ajoute 1,257 kg d'éthanol et chauffe à 50°C jusqu'à dissolution complète. Le mélange est filtré à chaud, puis, au filtrat, on ajoute en 1 heure 4,39 kg d'eau purifiée en maintenant la température à 50°C. Après avoir amorcé la cristallisation, le mélange est refroidi à 0°C en 4 heures. Les cristaux sont séparés par filtration, lavés avec 0,909 kg puis 0,606 kg d'un mélange éthanol-eau (1-2 en poids) puis séchés à 38°C sous pression réduite (5,07 kPa) dans une atmosphère à 80 % d'humidité relative pendant 48 heures. On obtient ainsi 266,5 g de trihydrate de (2R,3S)-3-tert-butoxycarbonylamino-2-hydroxy-3-phénylpropionate de 4-acétoxy-2α-benzoyloxy-5β,20-époxy-1,7β,10β-trihydroxy-9-oxo-tax-11-èn-13a-yle dont l'analyse montre que son titre par chromatographie liquide à haute performance est de 98,7 % (sur sec) et que la teneur en eau est de 6,15 %.

### EXEMPLE 2

On dissout, à une température voisine de 35°C, 110,0 g de (2R,3S)-3-tert-butoxycarbonylamino-2-hydroxy-3-phénylpropionate de 4-acétoxy-2α-benzoyloxy-5β,20-époxy-1,7β,10β-trihydroxy-9-oxo-tax-11-èn-13α-yle titrant 92,5 % et 0,2224 g d'acide ascorbique dans 1340 cm3 d'éthanol. On distille environ 70 % de l'éthanol introduit sous pression réduite (8 kPa) à une température voisine de 20°C. Le mélange chauffé à 50°C puis filtré. Le filtre est lavé par 3 fois 70,5 cm3 d'éthanol puis on ajoute en 15 minutes 860,5 cm3 d'eau purifiée à 50°C. On ensemence avec quelques cristaux de trihydrate de (2R,3S)-3-tert-butoxycarbonylamino-2-hydroxy-3-phénylpropionate de 4-acétoxy-2α-benzoyloxy-5β,20-époxy-1,7β,10β-trihydroxy-9-oxo-tax-11-èn-13α-yle puis on agite pendant 30 minutes. On ajoute alors en 3 heures 860,5 cm3 d'eau purifiée à 50°C puis on refroidit en 3 heures jusqu'à une température voisine de 0°C. La bouillie est alors filtrée. Le gâteau de filtration est lavé par 330 g d'un mélange eau-éthanol (2-1 en poids) puis par 220 g du même mélange puis séché sous pression réduite (5 kPa) à 38°C dans une atmosphère à 80 % d'humidité relative. On obtient ainsi, avec un rendement de 98 %, 110,2 g de trihydrate de (2R,3S)-3-tert-butoxycarbonylamino-2-hydroxy-3-phénylpropionate de 4-acétoxy-2α-benzoyloxy-5β,20-époxy-1,7β,10β-trihydroxy-9-oxo-tax-11-èn-13α-yle.

## Revendications

1. Procédé de préparation du trihydrate de (2R,3S)-3-tert-butoxycarbonylamino-2-hydroxy-3-phénylpropionate de 4-acétoxy-2α-benzoyloxy-5β,20-époxy-1,7β,10β-trihydroxy-9-oxo-tax-11-èn-13α-yle caractérisé en ce que l'on cristallise le (2R,3S)-3-tert-butoxycarbonylamino-2-hydroxy-3-phénylpropionate de 4-acétoxy-2α-benzoyloxy-5β,20-époxy-1,7β,10β-trihydroxy-9-oxo-tax-11-èn-13α-yle dans un mélange d'eau et d'un alcool aliphatique contenant 1 à 3 atomes de carbone, puis sèche le produit obtenu dans des conditions déterminées de température, de pression et d'humidité.

2. Procédé selon la revendication 1 caractérisé en ce que le rapport pondéral eau/alcool est voisin de 2/1.

3. Procédé selon l'une des revendications 1 ou 2 caractérisé en ce que l'alcool est l'éthanol.

4. Procédé selon la revendication 1 caractérisé en ce que le séchage est effectué à un température voisine de 40°C, sous une pression comprise entre 4 et 7 kPa et dans une atmosphère dont l'humidité relative est voisine de 80 %.

5. Procédé selon la revendication 1 caractérisé en ce que la cristallisation est effectuée en présence d'acide ascorbique.

## Patentansprüche

1. Verfahren zur Herstellung des Trihydrates von (2R,3S)-3-tert.-Butoxycarbonylamino-2-hydroxy-3-phenylpropionat von 4-Acetoxy-2α-benzoyloxy-5β,20-epoxy-1,7β,10β-trihydroxy-9-oxo-tax-11-en-13α-yl, dadurch gekennzeichnet, daß man das (2R,3S)-3-tert.-Butoxycarbonylamino-2-hydroxy-3-phenylpropionat von 4-Acetoxy-2α-benzoyloxy-5β,20-epoxy-1,7β,10β-trihydroxy-9-oxo-tax-11-en-13α-yl in einer Mischung von Wasser und einem aliphatischen Alkohol mit 1 bis 3 Kohlenstoffatomen kristallisiert und anschließend das erhaltene Produkt unter bestimmten Bedingungen von Temperatur, Druck und Feuchtigkeit trocknet.

2. Verfahren nach Anspruch 1, dadurch gekennzeichnet, daß das Gewichtsverhältnis Wasser/Alkohol etwa 2/1 beträgt.

3. Verfahren nach einem der Ansprüche 1 oder 2, dadurch gekennzeichnet, daß der Alkohol Ethanol ist.

4. Verfahren nach Anspruch 1, dadurch gekennzeichnet, daß die Trocknung bei einer Temperatur von etwa 40 °C, unter einem Druck zwischen 4 kPa und 7 kPa und in einer Atmosphäre durchgeführt wird, deren relative Feuchte etwa 80 % beträgt.

5. Verfahren nach Anspruch 1, dadurch gekennzeichnet, daß die Kristallisation in Anwesenheit von Ascorbinsäure durchgeführt wird.

## Claims

1. Process for the preparation of 4-acetoxy-2α-benzoyloxy-5β,20-epoxy-1,7β,10β-trihydroxy-9-oxotax-11-en-13α-yl (2R,3S)-3-tert-butoxycarbonylamino-2-hydroxy-3-phenylpropionate trihydrate characterized in that the 4-acetoxy-2α-benzoyloxy-5β,20-expoxy-1,7β,10β-trihydroxy-9-oxotax-11-en-13α-yl(2R,3S)-3-tert-butoxycarbonylamino-2-hydroxy-3-phenylpropionate is crystallized from a mixture of water and an aliphatic alcohol containing 1 to 3 carbon atoms, and the product obtained is then dried under defined conditions of temperature, pressure and humidity.

2. Process according to claim 1, characterised in that the water/alcohol weight ratio is close to 2/1.

3. Process according to either of claims 1 or 2 characterized in that the alcohol is ethanol.

4. Process according to claim 1, characterized in that the drying is performed at a temperature close to 40°C, at a pressure of between 4 and 7 kPa and in an atmosphere whose relative humidity is close to 80%.

5. Process according to claim 1, characterised in that the crystallisation is performed in the presence of ascorbic acid.
